# EUROPEAN PATENT SPECIFICATION

(11) **EP 2 967 946 B1**
(45) Date of publication and mention of the grant of the patent: **01.03.2023**
(21) Application number: 14770194.0
(22) Date of filing: 14.03.2014
(51) Int. Cl.: A61F 2/954, A61M 25/09

(54) **AUXILIARY SMALL VASCULATURE GUIDEWIRE**
ZUSATZFÜHRUNGSDRAHT FÜR KLEINE GEFÄSSE
FIL-GUIDE DE PETIT SYSTÈME VASCULAIRE AUXILIAIRE

(30) Priority: 14.03.2013 US 201361783023 P
(43) Date of publication of application: 20.01.2016
(73) Proprietor: Millett, Bret, San Diego, CA 92130 (US); Burnett, Joseph, San Diego, CA 92130 (US)
(72) Inventor: Millett, Bret, San Diego, CA 92130 (US); Burnett, Joseph, San Diego, CA 92130 (US)
(74) Representative: Philips Intellectual Property & Standards
(86) International application number: PCT/US2014/027230
(87) International publication number: WO 2014/152340

(56) References cited:
- WO-A1-2007/057132
- WO-A1-2009/046097
- US-A1- 2001 049 548
- US-A1- 2002 183 763
- US-A1- 2006 100 694
- US-A1- 2007 112 331
- US-A1- 2007 208 276
- US-A1- 2010 056 985
- US-A1- 2010 292 614

## Description

### Field of the Invention

The invention generally relates to auxiliary guidewires for intravascular procedures that operate with guidewires for intravascular procedures.

### Background

Some people are at risk of having a heart attack or stroke due to fatty plaque buildups in their arteries that restrict the flow of blood or even break off and block the flow of blood completely. Angioplasty is a procedure for treating sites that are affected by plaque. In this procedure, a needle is used to make an opening through a patient's skin. A guidewire is then inserted through the hole and guided through an artery and to the affected site. The physician tries to guide the wire by twisting and manipulating the proximal end that sits outside the patient.

The guidewire is meant to help in a number of treatment options. For example, an imaging guidewire (e.g., with an ultrasound or optical imaging sensor) can be used to visualize the affected site. If the affected blood vessel is severely narrowed by plaque buildup, the guidewire can be used to deliver, via a catheter, a balloon or stent to the affected site in hopes of opening up the narrowed vessel. If the affected site is totally occluded, the guidewire or a specialized tool can be used to cut through the occlusion.

A number of problems are associated with these procedures. For example, blockage may occur in vasculature that is too fine for a standard guidewire. A guidewire fine enough to reach such vessels may be too floppy to use. Branched vessels also present navigational challenges. It can be difficult to guide a wire into the correct branch.

WO 2007/057132 A1 describes an elongate device for introduction into a body lumen comprises a body extending in a longitudinal direction, and having at least two skeletal members, which are substantially aligned in the longitudinal direction, and at least one electroactive polymer material, which changes volume upon electrical activation, arranged to control a distance between two longitudinally spaced-apart portions of aid skeletal members. The body presents an asymmetric bending stiffness, and/or the electroactive polymer material is asymmetrically arranged about a central axis of the device, such that the body is arranged to bend transversely of the longitudinal direction upon activation of the electroactive polymer material.

US 2007/0112331 A1 describes medical devices which may include structure or provision that permit a physician or other health care professional to adjust particular parameters such as flexibility, stiffness and compressive strength of at least a portion of the medical device.

US 2010/0292614 describes medical systems, devices and methods for coupling a wire guide to a previously introduced wire guide in intracorporeal procedures, and generally include a supplemental wire guide and a tracking element. The tracking element defines a first passageway and a second passageway. The first passageway is sized to receive the previously introduced wire guide and the second passageway is sized to receive the supplemental wire guide. The supplemental wire guide is attached to the tracking element in a coupled configuration and detached from the tracking element in a decoupled configuration for independent translation of the supplemental wire guide.

WO2009046097 A1 relates to coupling a wire guide to a previously introduced wire guide for assistance during interventional procedures in vessels with proximal tortuosity, or as a more substantial wire guide for angioplasty procedures, stenting procedures, and other device placement procedures and their related devices.

### Summary

The invention, as defined in independent claim 1 and subsequent dependent claims 2 - 10, provides an auxiliary guidewire with a sleeve member that slips over a primary guidewire and can provide a very fine extension beyond the end of the primary guidewire. The primary guidewire can be taken to its limit within the fine vasculature, and the auxiliary guidewire can then extend further into the fine vasculature. The primary guidewire can be taken to a bifurcation. The primary guidewire and the auxiliary guidewire can be sent down separate branches of the bifurcation. A physician can select which of the primary guidewire and the auxiliary guidewire are within the branch that needs to be treated by, for example, consulting an angiography display. The other guidewire can be removed, and the treatment can be delivered by catheter to the correct branch of the bifurcation. The auxiliary guidewire slips over the primary guidewire by means of a sleeve member. The inventive sleeve member is loosened or contracted by including an electroactive polymer in the sleeve member. This allows the auxiliary guidewire to alternatively be moved along with, or moved along relative to, the primary guidewire. Using an auxiliary guidewire as a very fine extension of a primary guidewire thus gives a physician tools for navigating the very fine vasculature of a patient. Fatty plaque buildups that otherwise could not be treated can be treated.

The invention provides an auxiliary guidewire comprising at least one sleeve member disposed along an extended body. The sleeve member preferably defines an aperture area of about 0,035cm (0.014"). The auxiliary guidewire preferably is a sub-0,035cm (sub-0.014") auxiliary guidewire. The sleeve member includes at least one section of electroactive polymer. The auxiliary guidewire may include a plurality of sleeve members. A sleeve member may include a skive.

Provided is an exemplary system for treating the fine vasculature, the system including a guidewire, a catheter, and an auxiliary guidewire. The guidewire is preferably a standard 0,035cm (0.014") guidewire. And the auxiliary guidewire includes at least one sleeve member disposed along an extended body having an aperture area of about 0,035cm (0.014"). The auxiliary guidewire preferably is a sub-0,035cm (sub-0.014") auxiliary guidewire. The sleeve member may include at least one section of electroactive polymer.

Further provided is a method, not forming part of the invention, of inserting a catheter into a selected branch of a bifurcation. The method includes viewing the bifurcation on an angiographic display.

A select branch of the bifurcation is identified for treatment. A guidewire is inserted up to the bifurcation. An auxiliary guidewire is slid over the guidewire and brought to the branch.

The auxiliary guidewire is sent down one branch and the guidewire is sent down the other branch. Which of the auxiliary guidewire and the guidewire is within the select branch is identified by the angiographic display. The other of the auxiliary guidewire and the guidewire is removed. A catheter is delivered to the select branch.

### Brief Description of the Drawings

FIG. 1 shows a catheter with a guidewire.
FIG. 2 shows a guidewire.
FIG. 3 shows an auxiliary guidewire of the invention.
FIG. 4 illustrates a system including an auxiliary guidewire.
FIG. 5 shows a skive on a sleeve of the auxiliary guidewire.
FIG. 6 presents a skive on an auxiliary guidewire.
FIG. 7 depicts use of an auxiliary guidewire to access fine vasculature.
FIG. 8 shows use of a system of the invention to choose a branch of a bifurcation.

### Detailed Description

The invention, as defined in independent claim 1 and subsequent dependent claims 2 - 10, provides an auxiliary guidewire for an intravascular procedure that includes one or more sleeve or skive made with an electroactive polymer at one or a number of locations along the auxiliary guidewire. The auxiliary guidewire is a sub-0,035cm (sub-0.014") guidewire and can be inserted onto and slid along a standard (e.g., 0,035cm (0.014")) guidewire. The guidewire, the auxiliary guidewire, or both can be used in an intravascular procedure, such as crossing a chronic total occlusion or delivering a balloon or stent in a coronary angioplasty procedure.

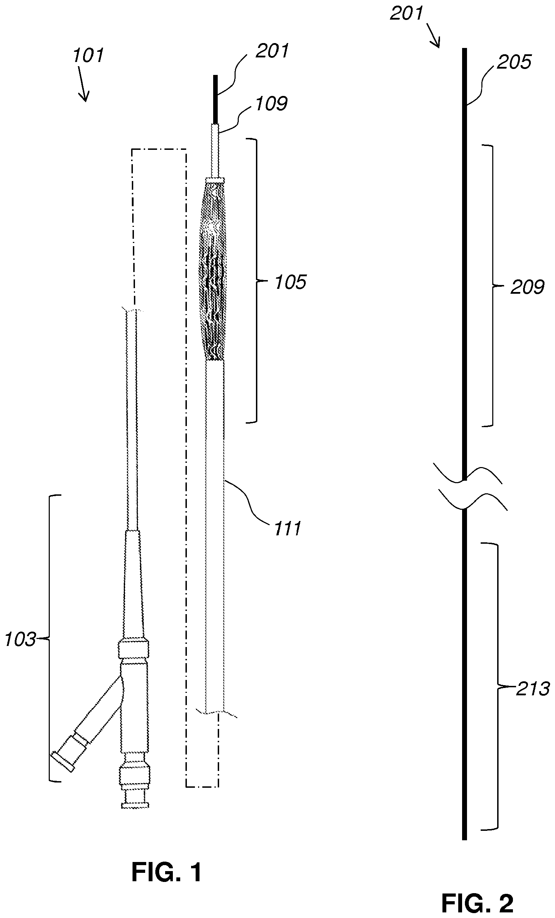

FIG. 1 shows a catheter 101 with a guidewire 201 disposed therethrough. Catheter 101 generally includes a proximal portion 103 extending to a distal portion 111. Optionally, a therapeutic device 105, such as a balloon or stent, may be located near distal tip 109.

FIG. 2 shows guidewire 201 including a proximal portion 213 extending to a distal portion 209 and terminating at distal tip 205. Guidewire 201 is preferably a standard 0,035cm (0.014") guidewire.

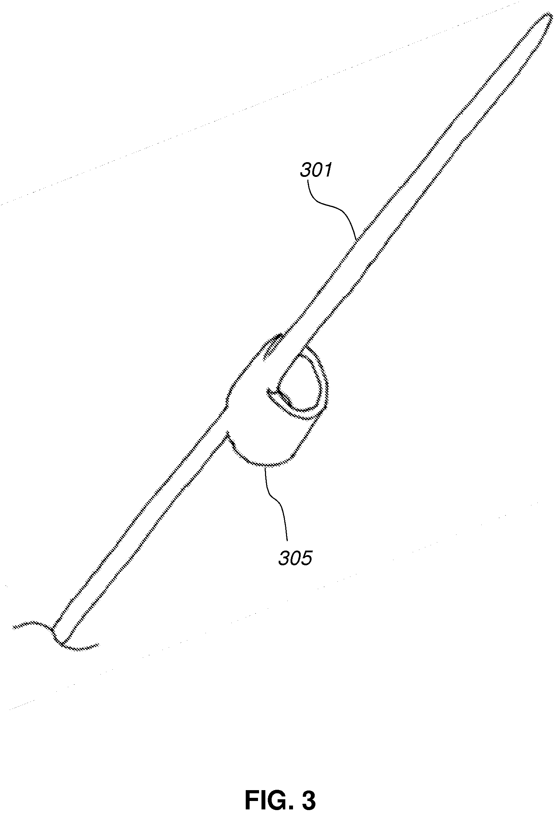

FIG. 3 shows a distal portion of an auxiliary guidewire 301. Auxiliary guidewire 301 may have any suitable cross-sectional shape such as, for example, round, flat, oval, or kidney shaped in cross-section. Auxiliary guidewire includes one or more of a sleeve 305 dimensioned to be slid over guidewire 201. Sleeve member 305 includes an electroactive polymer. Auxiliary guidewire 301 is preferably a sub-0,035cm (sub-0.014") guidewire and can be slid onto a standard guidewire 201 at the proximal end through the use of one or more sleeve member 305.

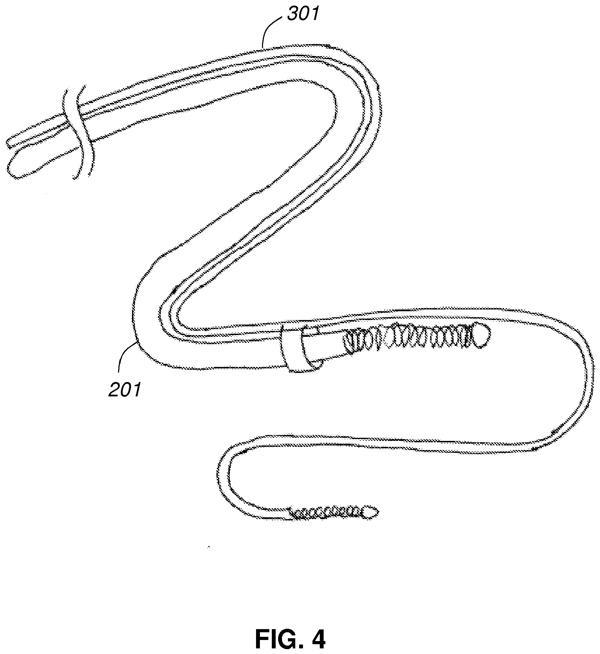

FIG. 4 shows an auxiliary guidewire 301 mated to a guidewire 201.

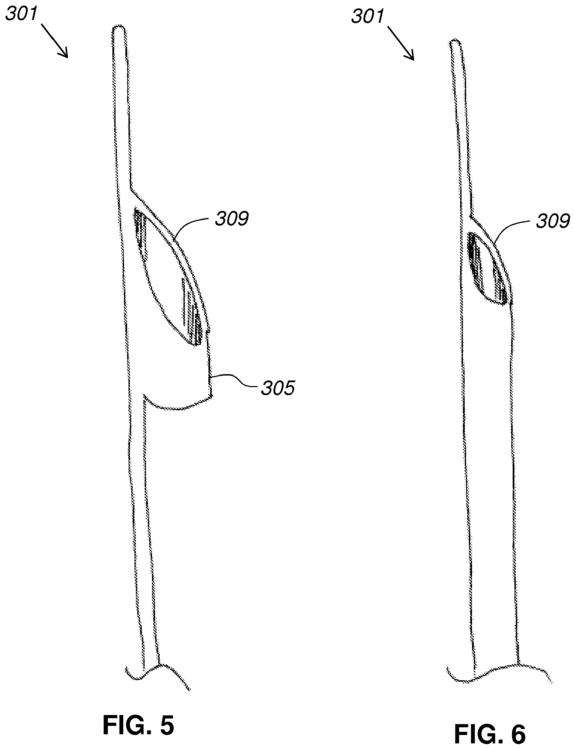

FIG. 5 depicts an alternative embodiment in which sleeve member 305 includes a skive 309. The inclusion of skive 309 may be beneficial to provide a slope for navigating into fine vasculature. Additionally, the inclusion of skive 309 may be beneficial by increasing an area of aperture into sleeve 305 making it easier to accomplish mating auxiliary guidewire 301 to guidewire 201.

FIG. 6 depicts an embodiment in which auxiliary guidewire 301 bears an extended lumen terminating at skive 309. This embodiment may be preferred where it is desired to keep auxiliary guidewire 301 substantially against guidewire 201.

The inventive auxiliary guidewire 301 includes at least one section of electroactive polymer. The inventive sleeve member 305 includes at least one section of electroactive polymer. The electroactive polymer causes sleeve 305 to deform (means , contract or expand) when energized. Sleeve member 305 can thus be expanded allowing insertion and sliding onto guidewire 201. Sleeve member 305 can be retracted or restricted to fix auxiliary guidewire 301 onto guidewire 201. Additionally, the electroactive polymer can be retracted or restricted to shrink sleeve member 305 after removing guidewire 201. Shrinking sleeve member 305 allows passage of a catheter 101 over auxiliary guidewire 201.

Electroactive polymers deform in the presence of an applied electric field, much like piezoelectric actuators. EAPs produce force, strain, deflections, or combination thereof. In general, types of EAPs include ionic, dielectric, and composites. The ionic EAPs operate through the movement of ions within a polymer. The ionic EAPs have the potential of matching the force and energy density of biological muscles. Ionomeric polymer-metal composites (IPMC) are electroactive polymers that bend in response to an electrical activation as a result of the mobility of cations in the polymer network. Generally, two types of base polymers are employed to form IPMCs such as perfluoro sulphonate sold under the trademark NAFION by Du Pont and perfluorocaboxylate sold under the trademark FLEMION by Asahi Glass, Japan. IPMC require relatively low voltages to stimulate a bending response (1-10 V) with low frequencies below 1 Hz.

Certain crystals (e.g. quartz, tourmaline and Rochelle salt), when compressed along certain axes, produced a voltage on the surface of the crystal. The reverse effect is also exhibited, whereby application of an electric current deforms the crystal. Any suitable electroactive material may be included. Suitable materials include poly(vinylidene fluoride) or PVDF and its copolymers. These materials include a partially crystalline component in an inactive amorphous phase. Applied AC fields (∼200 MV/m) induce electrostrictive (non-linear) strains of about 2%. P(VDF-TrFE) is a PVDF polymer that has been subject to electron radiation and has shown electrostrictive strain as high as 5% at lower frequency drive fields (150 V/mm).

Electrostatic fields can be employed to those polymers exhibiting low elastic stiffness and high dielectric constants to induce large actuation strain, these polymers are known as electrostatically stricted polymers (ESSP) actuators.

Ferroelectric electroactive polymer actuators can be operated in air, vacuum or water and throughout a wide temperature range.

Dielectric electroactive polymers are essentially an elastomeric capacitor. Electrostatic forces cause charged electrodes to compress an intermediate polymer layer, causing a strain response such as an expansion in a direction orthogonal to the compression. The process is also reversible, which can be used to generate electricity or be used as a sensor (much like piezoelectrics). Dielectric electroactive polymers form the basis of the electroactive polymer artificial muscle (EPAM) "spring roll" actuators. Dielectric electroactive polymer actuators can use large electric fields (~100 V/mm) and can produce strain levels (10-200%). An acrylic elastomer tape such as the tape sold under the trademark VHB by 3M is capable of planar strains of more than 300% for biaxially symmetric constraints and linear strains up to 215% for uniaxial constraints.

Electrostrictive graft elastomers include two components, a flexible macromolecule backbone and a grafted polymer that can be produced in a crystalline form. The material exhibits high electric field induced strain (~4%) combined with mechanical power and excellent processability. In some embodiments, the invention provides an electrostrictive-grafted elastomer with a piezoelectric poly(vinylidene fluoride-trifluoro-ethylene) copolymer. This combination has the ability to produce a varied amount of ferroelectric-electrostrictive molecular composite systems. These may be operated as a piezoelectric sensor or even an electrostrictive actuator.

Embodiments of the invention can include electro-viscoelastic elastomers that comprise a silicone elastomer and a polar phase. Upon curing, an electric field is applied that orientates the polar phase within the elastomeric matrix. An applied electric field (<6 V/mm) induces changes in shear modulus.

Liquid crystal elastomer (LCE) materials possess electroactive polymer characteristics by inducing Joule heating. LCEs are composite materials consisting of monodomain nematic liquid crystal elastomers and conductive polymers, which are distributed within their network structure. The actuation mechanism is a phase transition between nematic and isotropic phases. The actuation takes place in less than a second.

Conductive polymers (CP) includes EAPs that actuate via the reversible counter-ion insertion and expulsion that occurs during redox cycling. Significant volume changes occur through oxidation and reduction reactions at corresponding electrodes through exchanges of ions with an electrolyte. Conducive polymer actuators requires voltages in the range of 1-5 V. Variations to the voltage can control actuation speeds. Relatively high mechanical energy densities of over 20 J/cm³ are attained with these materials. Electrodes for conductive polymers may be fabricated from polypyrrole or polyaniline, or PAN doped with HCl. Other material combinations for conductive polymers are polypyrrole, polyethylenedioxythiophene, poly(p-phenylene vinylene)s, polyaniline and polythiophenes.

Carbon Nanotubes (CNT) are polymers that can be actuated via an electrolyte medium and the change in bond length via the injection of charges that affect the ionic charge balance between the nano-tube and the electrolyte. The more charges that are injected into the CNT the larger the dimension change. Due to the mechanical strength and modulus of single CNTs and the achievable actuator displacements, these electroactive polymers can boast the highest work per cycle and generate much higher mechanical stresses than other forms of electroactive polymers.

The inclusion of the electroactive polymer can be used to influence one or more other properties of auxiliary guidewire 301. Any property associated with a dimensional change in response to an applied potential may be included. Exemplary properties include variable stiffness due to the inclusion of at least one section of electroactive polymer at one or more different locations on auxiliary guidewire 301.

In certain examples, actuation of the electroactive polymer causes the region surrounding the electroactive polymer section to increase in stiffness, thereby increasing the pushability of auxiliary guidewire 301. Alternatively, actuation of the at least one section of electroactive polymer causes the region surrounding the electroactive polymer section to decrease in stiffness, thereby increasing flexibility of auxiliary guidewire 301.

In one example, the at least one section of electroactive polymer forms part of either the inner or outer shaft of auxiliary guidewire 301. In one example, the at least one section of electroactive polymer is a longitudinal strip. In one example, a shaft of auxiliary guidewire 301 is manufactured of electroactive polymer. In one example, the at least one section of electroactive polymer forms the outer surface of the inner shaft. In one example, the at least one section of electroactive polymer is located in a tip of auxiliary guidewire 301.

An electroactive polymer can provide an ability to curve or turn, for example, to navigate the vasculature system due to strategic positioning of at least one section of electroactive polymer at different locations on the guidewire. In one example, at least one section of electroactive polymer is located only on one side of the inner shaft to control the deflection of the distal tip. In one example, at least one section of electroactive polymer changes the spatial configuration of the guidewire to improve steering around corners. In one example, the guidewire has at least one section of electroactive polymer. In one example, the guidewire tip has at least one section of electroactive polymer. In one example, the at least one section of electroactive polymer in an actuated state causes the guidewire to contract axially. Motions that can be exhibited by auxiliary guidewire 301 include stretching or compression, axial rotation (e.g., torque), lateral vibration, reciprocation (e.g., sawing or toothbrush motion), or any others, or a combination thereof.

In addition to improved navigation through the inclusion of electroactive polymer, auxiliary guidewire 301 exhibits improved navigation by virtue of its small diameter. For example, auxiliary guidewire 301 may be inserted into a vessel into which guidewire 201 will not fit.

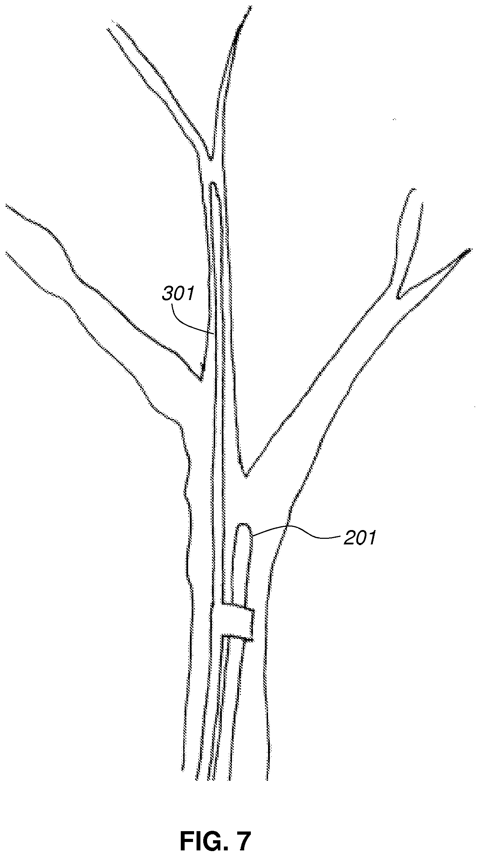

FIG. 7 illustrates a navigational advantage provided by auxiliary guidewire 301. Inserting guidewire 201 provides a path for auxiliary guidewire 301 to follow. Auxiliary guidewire 301 provides a means for navigating further down the vasculature than is possible with only guidewire 201.

The fine vasculature navigability of auxiliary guidewire 301 in combination with the improved dexterity of auxiliary guidewire 301 provide further navigational advantages. For example, due to the inclusion of at least one electroactive polymer, auxiliary guidewire 301 by be turned to enter an off-axis branch.

Further, auxiliary guidewire 301 may bifurcate from guidewire 201 at a point proximal from a distal tip of guidewire 201 (e.g., auxiliary guidewire 301 and guidewire 201 form a "Y" shape). As a result, a physician can navigate into both branches of a bifurcation in a vessel.

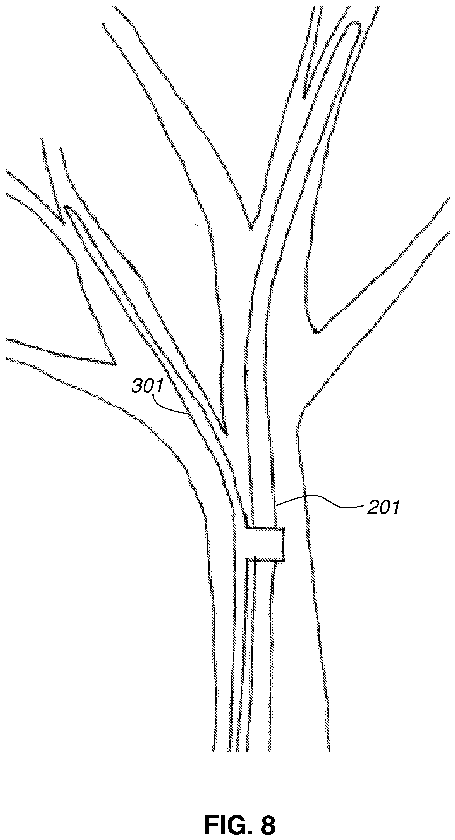

FIG. 8 illustrates use of auxiliary guidewire 301 to navigate into paths that branch off from the vasculature tracked by guidewire 201. Navigating a bifurcation is known in the art to be a difficult challenge. See, e.g., Suzuki, et al., 2013, A novel guidewire approach for handling acute-angle bifurcations, J Inv Cardiol 25(1):48-54; Lefevre, et al., 2007, Stenting of bifurcation lesions, J Interven Cardiol 14(6):573-585; and Oesterle, et al., 2005, Angioplasty at coronary bifurcations, Cath Card Diag 12(1):57 -63.

Where a bifurcated vessel must be treated, the bifurcation shown in FIG. 8 may be obtained. The physician may then view the disposition of auxiliary guidewire 301 and guidewire 201 (e.g., via angiography) and determine which branch of the bifurcation to send the treatment catheter to. Whichever branch the physician determines to treat, the guidewire corresponding to the other branch can then be removed. Thus an auxiliary guidewire 301 gives the ability to select which branch of a bifurcated vessel to send a catheter to. Bifurcation is discussed in U.S. Pat. 8,088,102 to Adams; U.S. Pat. 7,300,460 to Levine; U.S. Pub. 2009/0326634 to Vardi; and U.S. Pub. 2001/0049548 to Vardi.

Due to the fact that curvature of auxiliary guidewire 301 can be induced from a computer workstation (e.g., by a mouse, joystick, or computer keys), auxiliary guidewire 301 can be navigated through or into vessels, even where tortuous or branched. For example, a physician may refer to an angiographic display. Angiography systems can be used to visualize the blood vessels by injecting a radio-opaque contrast agent into the blood vessel and imaging using X-ray based techniques such as fluoroscopy.

Angiographic techniques include projection radiography as well as imaging techniques such as CT angiography and MR angiography. In certain embodiments, angiography involves using an x-ray contrast agent and an x-ray system to visualize the arteries and guidewire 201. X-ray images of the transient radio contrast distribution within the blood flowing within the coronary arteries allows visualization of the location of guidewire 201, particularly in relation to the artery openings.

A physician may refer to the angiography display to navigate guidewire 201. Angiography systems and methods are discussed, for example, in U.S. Pat. 7,734,009; U.S. Pat. 7,564,949; U.S. Pat. 6,520,677; U.S. Pat. 5,848,121; U.S. Pat. 5,346,689; U.S. Pat. 5,266,302; U.S. Pat. 4,432,370; and U.S. Pub. 2011/0301 684.

Useful catheters and guidewires are discussed in U.S. Pat. 7,766,896 and U.S. Pat. 7,909 844.

Auxiliary guidewire 301 may include a size adjustment mechanism to adjust the circumferential size of the guidewire. In the example, the size adjustment mechanism may operate through a pair of electroactive polymer actuators. The electroactive polymer actuators are configured to undergo deflection upon actuation to adjust the circumferential size of the guidewire.

In general, auxiliary guidewire 301 may include one or more electroactive polymer actuator with an elastomeric polymer positioned between a pair of electrodes. The elastomeric polymer layer may be configured to deflect when a voltage difference is applied across the elastomeric polymer layer. The electroactive polymer actuator can include one or more of any of a number of polymers, including, for example, dielectric electrostrictive electroactive polymers, ion-exchange electroactive polymers, and ionomeric polymer-metal composite electroactive polymers. For certain implementations, dielectric electrostrictive electroactive polymers are particularly desirable because of their response times and operational efficiencies. Specific examples of polymers that can be used include Nusil CF19-2186 (available from Nusil Technology, Carpenteria, Calif.); dielectric elastomeric polymers; silicone rubbers; silicone elastomers; acrylic elastomers, such as VHB 4910 acrylic elastomer (available from 3M Corporation, St. Paul, Minn.); silicones, such as Dow Corning HS3 (available from Dow Corning, Wilmington, Del.); fluorosilicones, such as Dow Corning 730 (available from Dow Corning, Wilmington, Del.); acrylic polymers, such as acrylics in the 4900 VHB acrylic series (available from 3M Corporation, St. Paul, Minn.); polyurethanes; thermoplastic elastomers; copolymers including poly(vinylidene fluoride); pressure-sensitive adhesives; fluoroelastomers; polymers including silicone and acrylics, such as copolymers including silicone and acrylic and polymer blends including a silicone elastomer and an acrylic elastomer; and combinations of two or more of these polymers. Electroactive polymers are discussed in U.S. Pat. 8,206,429; U.S. Pat. 8,133,199; U.S. Pat. 6,514,237; U.S. Pat. 5,573,520; U.S. Pat. 4,830;023; U.S. Pub. 2012/0265268; and U.S. Pub. 2007/0208276. Use of electroactive polymers is discussed further in U.S. Pat. 8,100,838; U.S. Pat. 8,021,377; U.S. Pat. 6,969,395; U.S. Pat. 6,139,510; U.S. Pub. 2005/0165439; and U.S. Pub. 2004/0220606.

In some examples, at least one section of electroactive polymer forms a spiral about auxiliary guidewire 301. The spiral may be, for example, a single, multiple sections of electroactive polymer or one continuous section of electroactive polymer. In at least one example, there are several sections of electroactive polymer which form an overall spiral pattern. In at least one example, the at least one section of electroactive polymer extends substantially the entire length of the guidewire in a spiral pattern. A spiral section of electroactive polymer can be selectively actuated to cause forced curvature or straightening of auxiliary guidewire 301. For example, after auxiliary guidewire 301 is deployed in a vessel and has been used, it may lie in a curved shape which could interfere with, for example, a deployed stent while the guidewire is being withdrawn. In at least one example, selective actuation will resist or prevent the inner shaft from holding, adopting, or maintaining the curvature or shape of a vessel during withdrawal of the guidewire.

As discussed herein, the actuation of the electroactive polymer improves the steering of the guidewire around corners or turns as the guidewire traverses the vasculature.

The auxiliary guidewire 301 can be manufactured by co-extruding a removable nylon wire in the wall of the guidewire shaft. After the nylon wire is pulled out, the resulting shaft can be coated with a conductive ink to form the electrode and filled with an electroactive polymer by electro polymerization. The counter electrode can be a conductive ink on the outside of the guidewire shaft. Each axial section of electroactive polymer may be deposited on one fraction of the circumference of a metallic auxiliary guidewire 301. A counter electrode can be deposited or printed on an insulator, which is positioned on auxiliary guidewire 301 opposite from the section of electroactive polymer. Actuation of the section of electroactive polymer causes auxiliary guidewire 301 to bend in a direction that is opposite from where the section of electroactive polymer coats auxiliary guidewire 301. Desirably, in use, these axial sections of electroactive polymer will allow the physician to control the direction of auxiliary guidewire 301 and allow for better maneuvering within the body lumen.

In at least one example, auxiliary guidewire 301 includes a polymer heat shrink tube made from polyester (PET). A conductive ink, for example, but not limited to, a silver or gold ink from Erconinc can be deposited onto the PET film. Because lines of conductive ink can be made very fine, multiple conductor lines can be printed along auxiliary guidewire 301. At the position of the electroactive polymer actuator, a larger surface can be printed and the electroactive polymer deposited.

Additionally or alternatively, an electroactive polymer can be used to stiffen or un-stiffen (e.g., make floppy) select portions of auxiliary guidewire 301. Auxiliary guidewire 301 may include a plurality of longitudinal strips of electroactive polymer positioned about the circumference of the guidewire shaft. Multiple strips of electroactive polymer, located at the same circumferential coordinate, may be positioned along the longitudinal length of the guidewire shaft. The exact placement about the circumference of the guidewire shaft is not critical so long as the strips of electroactive polymer are located about the entire circumference of the shaft along the area(s) where control of the flexibility/rigidity of the guidewire shaft is desired. Desirably, actuation of the longitudinal strips of electroactive polymer modifies the rigidity of the guidewire shaft in the region of the electroactive polymer strips. The strips may then be used to increase the stiffness and decrease the flexibility of the guidewire. In one example, the longitudinal strips decrease in size when actuated and decrease the stiffness and increase the flexibility of the guidewire. In one example, longitudinal strips of electroactive polymer are positioned about the circumference of the guidewire shaft and extend from the proximal end region of the guidewire shaft to the distal end region of the guidewire shaft. In addition, the number of strips of electroactive polymer positioned about the circumference of the guidewire shaft can vary. The actuator mechanism generally includes electrodes. The electrodes of different sections of electroactive polymer are separate from one another so that precise actuation of the desired section(s) of electroactive polymer can be done. An exterior surface of a strip of electroactive polymer may be substantially flush with the exterior surface of the guidewire shaft. In some examples, the strip of electroactive polymer may form only a portion of the wall of the guidewire shaft, i.e. the strip of electroactive polymer does not have the same thickness as the wall of the guidewire shaft and is not flush with either the exterior surface or the interior surface of the shaft.

In certain examples, stiff elements, e.g. stiff polymer strips, are engaged to a layer of electroactive polymer. If a guidewire 201 with greater stiffness is desired, the layer of electroactive polymer is actuated. Actuation of the layer of electroactive polymer causes the electroactive polymer to volumetrically increase in size and moves the stiff polymer strips outwards, to cause an increase in the stiffness of the guidewire 201 because the stiffness increases with the fourth power of the size. The polymer strips may extend along the entire length of the guidewire 201 or the strips may be positioned at particular areas along the length of the guidewire 201 where control of the stiffness of the guidewire shaft is desired. Similarly, the layer of electroactive polymer may extend along the entire length of the guidewire 201 or the layer of electroactive polymer may be placed at particular areas along the length of the guidewire 201 where control of the stiffness of the guidewire shaft is desired. In one example, at least one portion of the guidewire has a layer of electroactive polymer with at least one strip of stiff polymer engaged thereto. Examples of suitable materials to be used for the stiff polymer strips include, but are not limited to, polyamides, polyethylene (PE), Marlex high density polyethylene, polyetheretherketone (PEEK), polyamide (PI), and polyetherimide (PEI), liquid crystal polymers (LCP), acetal and any mixtures or combinations thereof. The polymer and actuators may be placed, for example, as described in U.S. Pub. 2005/0165439.

The parts of auxiliary guidewire 301 of the present invention may be manufactured from any suitable material to impart the desired characteristics and electroactive polymers. Examples of suitable materials include, but are not limited to, polymers such as polyoxymethylene (POM), polybutylene terephthalate (PBT), polyether block ester, polyether block amide (PEBA), fluorinated ethylene propylene (FEP), polyethylene (PE), polypropylene (PP), polyvinylchloride (PVC), polyurethane, polytetrafluoroethylene (PTFE), polyether-ether ketone (PEEK), polyimide, polyamide, polyphenylene sulfide (PPS), polyphenylene oxide (PPO), polysulfone, nylon, perfluoro(propyl vinyl ether) (PFA), polyether-ester, polymer/metal composites, etc., or mixtures, blends or combinations thereof. One example of a polyether block ester is available under the trade name ARNITEL, and one suitable example of a polyether block amide (PEBA) is available under the trade name PEBA, from ATOMCHEM POLYMERS, Birdsboro, PA.

The guidewires of the present invention are actuated, at least in part, using electroactive polymer actuators. Electroactive polymers are characterized by their ability to change shape in response to electrical stimulation. Electroactive polymers include electric electroactive polymers and ionic electroactive polymers. Piezoelectric materials may also be employed. Electric electroactive polymers include ferroelectric polymers, dielectric electroactive polymers, electrorestrictive polymers such as the electrorestrictive graft elastomers and electroviscoelastic elastomers, and liquid crystal elastomer materials.

Additional information regarding electroactive polymer actuators, their design considerations, and the materials and components that may be employed therein, can be found, for example, in U.S. Pat. 7,777399; U.S. Pat. 6,258,052; U.S. Pat. 6,249,076; U.S. Pat. 6,139,510; U.S. Pat. 5,693,015; U.S. Pat. 5,120,308; U.S. Pub. 2006/0100694; and U.S. Pub. 2006/0074442.

Furthermore, networks of conductive polymers may also be employed. For example, it has been known to polymerize pyrrole in electroactive polymer networks such as poly(vinylchloride), poly(vinyl alcohol), a perfluorinated polymer that contains small proportions of sulfonic or carboxylic ionic functional groups, available from E.I. DuPont Co., Inc. (Wilmington, DE). Electroactive polymers are also discussed in U.S. Pub. 2004/0143160 and U.S. Pub. 2004/006816 1.

As used herein, the word "or" means "and or or", sometimes seen or referred to as "and/or", unless indicated otherwise.

## Claims

1. A system for treating the fine vasculature, the system comprising
a primary guidewire (201);
an auxiliary guidewire (301) comprising:
an extended body; and
at least one sleeve member (305) disposed along the extended body and dimensioned to be slid over the primary guidewire,
**characterized in that** said sleeve member comprises at least one section of electroactive polymer that cause the sleeve member to contract so as to fix the auxiliary guidewire onto the primary guidewire or to expand when energized.

2. The system of claim 1, wherein the sleeve member (305) of the auxiliary guidewire (301) defines an aperture diameter of about 0,035cm (0.014").

3. The system of claim 1, wherein the auxiliary guidewire is a sub-0,035cm (sub-0.014") auxiliary guidewire.

4. The system of claim 1, wherein the auxiliary guidewire further comprises a plurality of sleeve members.

5. The system of claim 1, wherein the sleeve member of the auxiliary guidewire includes a skive (309).

6. The system of claim 1, the system further comprising:
a catheter comprising a lumen therethrough and having the primary guidewire extending through the lumen.

7. The system of claim 1, wherein the auxiliary guidewire (301) has a smaller diameter than a diameter of the primary guidewire, and extends beyond a distal end of the primary guidewire.

8. The system of claim 1, wherein the primary guidewire (201) is a standard 0,035cm (0.014") guidewire.

9. The system of claim 8, wherein the at least one sleeve member of the auxiliary guidewire (301) is disposed along an extended body having an aperture area of about 0,035cm (0.014").

10. The system of claim 9, wherein the auxiliary guidewire preferably is a sub-0,035cm (sub-0.014") auxiliary guidewire.

## Patentansprüche

1. System zur Behandlung der feinen Gefäße, wobei das System Folgendes umfasst
einen Primär-Führungsdraht (201);
einen Hilfsführungsdraht (301) mit:
einen verlängerten Körper; und
mindestens ein Hülsenelement (305), das entlang des verlängerten Körpers angeordnet und
so dimensioniert ist, dass es über den primären Führungsdraht geschoben werden kann,
**dadurch gekennzeichnet, dass** das Hülsenelement mindestens einen Abschnitt aus elektroaktivem Polymer umfasst, der
bewirkt,
Dass sich das Hülsenelement zusammenzieht, um den Hilfsführungsdraht auf dem primären Führungsdraht zu fixieren, oder sich ausdehnt, wenn es erregt wird.

2. System nach Anspruch 1, wobei das Hülsenelement (305) des Hilfsführungsdrahtes (301) einen Öffnungsdurchmesser von etwa 0,035 cm (0.014") definiert.

3. System nach Anspruch 1, wobei der Hilfsführungsdraht ein Hilfsführungsdraht unter 0,035 cm (unter 0.014") ist.

4. System nach Anspruch 1, wobei der Hilfsführungsdraht ferner eine Vielzahl von Hülsenelementen umfasst.

5. System nach Anspruch 1, wobei das Hülsenelement des Hilfsführungsdrahtes einen Skive (309) aufweist.

6. System nach Anspruch 1, wobei das System ferner Folgendes umfasst:
einen Katheter mit einem durchgehenden Lumen, durch das sich der primäre Führungsdraht erstreckt.

7. System nach Anspruch 1, wobei der Hilfsführungsdraht (301) einen kleineren Durchmesser als der Durchmesser des primären Führungsdrahtes hat und sich über ein distales Ende des primären Führungsdrahtes hinaus erstreckt.

8. System nach Anspruch 1, wobei der primäre Führungsdraht (201) ein 0,035 cm (0.014") Standard-Führungsdraht ist.

9. System nach Anspruch 8, wobei das mindestens eine Hülsenelement des Hilfsführungsdrahtes (301) entlang eines verlängerten Körpers angeordnet ist, der eine Öffnungsfläche von etwa 0,035 cm (0.014") aufweist.

10. System nach Anspruch 9, wobei der Hilfsführungsdraht vorzugsweise ein Hilfsführungsdraht von weniger als 0,035 cm (sub-0.014") ist.

## Revendications

1. Un système pour traiter le système vasculaire fin, le système comprend
un premier fil-guide (201);
un fil-guide auxiliaire (301) comprend :
un corps étendu; et
au moins un élément de manchon (305) disposé le long du corps étendu et dimensionné pour glisser sur le premier guide-fil, **caractérisé par le fait que** ledit élément de manchon comprend au moins une section de polymère électroactif qui fait en sorte que l'élément de manchon se contracte pour fixer le fil-guide auxiliaire sur le premier fil-guide ou se dilate sous tension.

2. Le système de la revendication 1, où l'élément de manchon (305) du fil-guide auxiliaire (301) définit un diamètre d'ouverture d'environ 0,035 cm (0,014").

3. Le système de la revendication 1, où le fil de guidage auxiliaire est un fil de guidage auxiliaire inférieur à 0,035 cm (inférieur à 0,014").

4. Le système de la revendication 1, où le guide-fil auxiliaire comprend une pluralité d'élément de manchons.

5. Le système de la revendication 1, où l'élément de manchon du guide-fil auxiliaire inclut une paroi (309).

6. Le système de la revendication 1 comprend également :
un cathéter comprenant une lumière qui le traverse et possède un premier guide-fil s'étendant à travers la lumière.

7. Le système de la revendication 1, où le guide-fil auxiliaire (301) possède un diamètre plus petit que le diamètre du premier guide-fil, et s'étend au-delà de l'extrémité distale du premier guide-fil.

8. Le système de la revendication 1, où le premier guide-fil (201) est un guide-fil standard 0,035 cm (0,014").

9. Le système de la revendication 8, où au moins un élément de manchon du guide-fil auxiliaire (301) est disposé le long d'un corps étendu possédant une zone d'ouverture d'environ 0,035 cm (0,014").

10. Le système de la revendication 9, où le guide-fil auxiliaire de préférence est un fil-guide auxiliaire de moins de 0,035 cm (moins de 0,014").
